# EUROPÄISCHE PATENTSCHRIFT

(11) **EP 1 735 343 B1**
(45) Veröffentlichungstag und Bekanntmachung des Hinweises auf die Patenterteilung: **29.10.2014**
(21) Anmeldenummer: 05716149.9
(22) Anmeldetag: 17.03.2005
(51) Int. Cl.: C07K 14/62

(54) **VERFAHREN ZUR GEWINNUNG VON INSULINEN DURCH VERBESSERTE LUFTBEGASUNG DER FALTUNG**
METHOD FOR EXTRACTING INSULINS BY IMPROVED AIR-GASSING OF A FOLDING
PROCEDE D'OBTENTION D'INSULINE PAR UNE MEILLEURE AERATION DU PLIAGE

(30) Priorität: 01.04.2004 DE 102004015965
(43) Veröffentlichungstag der Anmeldung: 27.12.2006
(73) Patentinhaber: Sanofi-Aventis Deutschland GmbH, 65929 Frankfurt am Main (DE)
(72) Erfinder: RUBROEDER, Franz-Josef, 65606 Villmar (DE); KELLER, Reinhold, 65812 Bad Soden (DE); HERBERT, Heike, 64579 Gernsheim (DE)
(86) Internationale Anmeldenummer: PCT/EP2005/002843
(87) Internationale Veröffentlichungsnummer: WO 2005/097827

(56) Entgegenhaltungen:
- EP-A- 0 668 292
- WO-A-01/46453

## Beschreibung

Die vorliegende Erfindung betrifft ein verbessertes Verfahren zur Gewinnung von Insulinen oder Insulinderivaten mit korrekt verbundenen Cystinbrücken in Gegenwart von Cystein oder Cysteinhydrochlorid und von einem chaotropen Hilfsstoff, wobei die Faltung in einem Reaktionsansatz erfolgt, bei dem das Verhältnis von Volumen zu Oberfläche größer als 1 und / oder die Sauerstoffkonzentration 1-15 mg/l ist.

Humaninsulin ist ein Protein mit zwei Aminosäureketten mit zusammen 51 Aminosäureresten. In den beiden Aminosäureketten kommen 6 Cysteinreste vor, wobei je zwei Cysteinreste über eine Disulfidbrücke untereinander verbunden sind. In biologisch aktivem Humaninsulin sind die A- und B-Ketten über zwei Cystinbrücken miteinander verbunden, und eine weitere Cystinbrücke kommt in der A-Kette vor. Innerhalb eines Humaninsulinmoleküls gibt es statistisch gesehen 15 Möglichkeiten zur Ausbildung von Disulfidbrücken. Im biologisch wirksamen Humaninsulin kommt nur eine der 15 Möglichkeiten vor. Die folgenden Cysteinreste sind im Humaninsulin miteinander verknüpft:
A 6-A 11
A 7-B 7
A20-B19

Die Buchstaben A und B stehen für die jeweilige Insulinaminosäurekette, die Zahl für die Position des Aminosäürerestes, die vom Amino- zum Carboxylende der jeweiligen Aminosäurekette gezählt wird. Disulfidbrücken können auch zwischen zwei Humaninsulinmolekülen ausgebildet werden, so daß leicht unübersehbar viele verschiedene Disulfidbrücken entstehen können.

Ein bekanntes Verfahren zur Herstellung von Humaninsulin beruht auf der Verwendung von humanem Proinsulin. Humanes Proinsulin ist ein Protein mit einer linearen Aminosäurekette aus 86 Aminosäureresten, wobei die B- und A-Ketten des Humaninsulins über ein C-Peptid mit 35 Aminosäureresten miteinander verbunden sind. Die Ausbildung der in Humaninsulin vorkommenden Disulfidbrücken erfolgt über ein Zwischenprodukt, wobei die Cysteinreste des Humaninsulins mit einer Schwefelschutzgruppe, z.B. einer S-Sulfonat (-S-SO₃⁻)-Gruppe versehen sind (EP 0 037 255). Ferner ist ein Verfahren zur Gewinnung von Proinsulin mit korrekt verbundenen Cystinbrücken bekannt (Biochemistry, 60, (1968), Seiten 622 bis 629), das von Proinsulin gewonnen aus Schweine-pankreas ausgeht, bei dem die Cysteinreste als Thiolreste (-SH) vorliegen. Unter dem Begriff "korrekt verbundenen Cystinbrücken" versteht man die Disulfidbrücken, die in biologisch aktivem Insulin aus Säugetieren vorkommen.

Gentechnische Verfahren erlauben es, Vorläufer von Insulin oder Insulinderivaten, im besonderen menschliches Proinsulin oder Proinsulin, das eine von Humaninsulin abweichende Aminosäuresequenz und/oder Aminosäurekettenlänge hat, in Mikroorganismen herzustellen. Die von gentechnisch veränderten Escherichia-coli-Zellen hergestellten Proinsuline haben keine korrekt verbundenen Cystinbrücken. Ein Verfahren zur Gewinnung von Humaninsulin mit E. coli (EP 0 055 945) beruht auf folgenden Verfahrensschritten:
Fermentation der Mikroorganismen - Zellaufschluß - Isolierung des Fusionproteins - Halogencyanspaltung des Fusionproteins - Isolierung des Spaltprodukts mit der Proinsulinsequenz - Schutz der Cysteinreste von Proinsulin durch S - Sulfonat-Gruppen - Chromatographische Reinigung.des S-Sulfonats - Ausbildung der korrekt verbundenen Cystinbrücken - Entsalzen des Proinsulins - Chromatographische Reinigung des Proinsulin mit korrekt verbundenen Cystinbrücken - Konzentrierung der Proinsulinlösung - Chromatographische Reinigung der konzentrierten Proinsulinlösung - Enzymatische Spaltung des Proinsulins zur Gewinnung von Humaninsulin - Chromatographische Reinigung des entstandenen Humaninsulins.

Nachteile dieses Verfahrens sind die Anzahl der Verfahrensschritte und die Verluste bei den Reinigungsschritten, die zu einer geringen Ausbeute an Insulin führen. Wegen des vielstufigen Verfahrensweges müssen erhebliche Verluste in Kauf genommen werden. Von der Stufe des isolierten Fusionproteins über Halogencyanspaltung, Sulfitolyse und Reinigung des Proinsulins muß mit einem bis zu 40%igem Verlust von Proinsulin gerechnet werden (EP 0 055 945). Ähnlich hohe Verluste können im Verlauf der nachfolgenden Reinigungsschritte bis zum Endprodukt auftreten. Ausbeutesteigerungen bei der gentechnischen Herstellung von Humaninsulin oder Insulinderivaten lassen sich dann erzielen, wenn die Zahl der notwendigen Verfahrensschritte wesentlich verringert werden kann.

Aus der EP 0 600 372 A1 (bzw. US 5,473,049) und der EP 0 668 292 A2 ist ein entsprechend verbessertes Verfahren zur Gewinnung von Insulinen oder Insulinderivaten bekannt, bei dem der Insulinvorläufer oder Vorläufer des Insulinderivates, dessen Cystinbrücken nicht korrekt verknüpft vorliegen, in Gegenwart eines Mercaptans, beispielsweise Cystein, und von mindestens einem chaotropen Hilfsstoff, beispielsweise Harnstoff oder Guanidinhydrochlorid, zu einem Insulinvorläufer bzw. Vorläufer des Insulinderivats mit korrekt verbundenen Cysteinbrücken umgesetzt wird. Bei dem bekannten Verfahren werden diese Proteine zunächst in wäßrigen Lösungen eines chaotropen Hilfsmittels oder von Mischungen von verschiedenen chaotropen Hilfsmitteln in sehr niedriger Konzentration gelöst. Anschließend wird die Proteinmischung mit einer wäßrigen Mercaptanlösung gemischt.

Überraschenderweise wurde nun gefunden, daß sich die Ausbeuten an korrekt gefalteten Vorläufern von Insulinen oder Insulinderivaten dadurch erhöhen und die Reaktionszeiten für den Faltungsprozeß dadurch verringern lassen, daß man den Vorläufer nicht in einem ersten Schritt mittels des chaotropen Hilfsstoffs in Lösung bringt, sondern daß man zunächst in die wäßrige Suspension des Vorläufers das Mercaptan, nämlich Cystein oder Cysteinhydrochlorid, einträgt und erst in einem nachfolgenden Schritt die Lösung des Vorläufers durch Eintrag in eine wäßrige Lösung des chaotropen Hilfsstoffs und schließlich die korrekte Faltung des Vorläufers durch Verdünnung des Gemisches auf eine bevorzugte Cystein- bzw. Cysteinhydrochloridkonzentration unter Eintrag des Gemisches in eine entsprechende Menge an Wasser bewirkt.

Demgemäß betrifft die vorliegende Erfindung ein Verfahren zur Gewinnung eines Vorläufers von Insulinen oder Insulinderivaten mit korrekt verbundenen Cystinbrücken in Gegenwart von Cystein oder Cysteinhydrochlorid und von einem chaotropen Hilfsstoff, dadurch gekennzeichnet, daß man nacheinander die folgenden Schritte durchführt:
(a) Versetzen einer wäßrigen Suspension des Vorläufers von Insulinen oder Insulinderivaten mit einer Menge an Cystein oder Cysteinhydrochlorid, die 1 bis 15 SH-Reste des Cysteins oder Cysteinhydrochlorids pro Cysteinrest des Vorläufers ergibt,
(b) Eintragen der cystein- oder cysteinhydrochloridhaltigen Suspension des Vorläufers in eine 4 bis 9 molare Lösung des chaotropen Hilfsstoffs bei einem pH-Wert von 8 bis 11,5 und einer Temperatur von 15 bis 55 °C, Halten des erhaltenen Gemischs für etwa 10 bis 60 Minuten auf dieser Temperatur und
(c) Eintragen des Gemischs bei einem pH-Wert von 8 bis 11,5 und einer Temperatur von 5 bis 30 °C in eine Menge an Wasser, welche eine Verdünnung der Konzentration des Cysteins oder des Cysteinhydrochlorids in dem Gemisch auf etwa 1 bis 5 mM und des chaotropen Hilfsstoffs auf 0,2 bis 1,0 M ergibt.
   wobei das Gemisch in Schritt (c) in einem Behälter begast wird, so dass die Sauerstoffkonzentration in dem Gemisch 1 bis 15 mg/l ist,
wobei das Verhältnis von Volumen zu Oberfläche des Gemisches größer als 1 m, insbesondere größer als 2 m, insbesondere größer als 3 m ist, und die Sauerstoffkonzentration in dem Gemisch bevorzugt 2 bis 10 mg/l beträgt.

Vorzugsweise ist das Verfahren auch dadurch gekennzeichnet, daß
in Schritt (a) die Menge an Cystein oder Cysteinhydrochlorid einer Menge entspricht, die 1 bis 6 SH-Reste des Cysteins oder Cysteinhydrochlorids pro Cysteinrest des Vorläufers ergibt,
in Schritt (b) das Eintragen der cystein- oder cysteinhydrochloridhaltigen Suspension des Vorläufers in eine 4 bis 9 molare Lösung des chaotropen Hilfsstoffs bei einem pH-Wert von 8 bis 11 und einer Temperatur von 30 bis 45 °C, das Halten des erhaltenen Gemischs für 20 bis 40 Minuten auf dieser Temperatur und
in Schritt (c) das Eintragen des Gemischs bei einem pH-Wert von 8 bis 11 und bei einer Temperatur von 15 bis 20 °C in eine Menge an Wasser, welche eine Verdünnung der Konzentration des Cysteins oder des Cysteinhydrochlorids in dem Gemisch auf etwa 1 bis 5 mM und eine Konzentration des chaotropen Hilfsstoffs von 0,2 bis 1,0 M ergibt, erfolgt.

Chaotrope Hilfsstoffe sind Verbindungen, die in wäßriger Lösung Wasserstoffbrückenbindungen brechen, beispielsweise Ammoniumsulfat, Guanidinhydrochlorid, Ethylencarbonat, Thiocyanat, Dimethylsulfoxid und Harnstoff.

Bei dem Verfahren gemäß der vorliegenden Erfindung wird als chaotroper Hilfsstoff bevorzugt Guanidin, Guanidinhydrochlorid oder besonders bevorzugt Harnstoff eingesetzt.

Die Konzentration des chaotropen Hilfsstoffs beträgt in Schritt (b) des erfindungsgemäßen Verfahrens vorzugsweise 7,0 bis 9,0 M, die Temperatur in Schritt (b) vorzugsweise 40°C und der pH-Wert in Schritt (b) vorzugsweise 10 bis 11.

Bei dem erfindungsgemäßen Verfahren beträgt der pH-Wert in Schritt (c) vorzugsweise 10 bis 11. In Schritt (c) des Verfahrens gemäß der vorliegenden Erfindung wird die Menge an Wasser, in welche das Gemisch eingetragen wird, vorzugsweise so gewählt, daß diese eine Verdünnung der Cystein- oder Cysteinhydrochloridkonzentration in dem Gemisch auf 2,5 bis 3,0 mM und eine Konzentration des chaotropen Hilfsstoffs von 0,5 M ergibt.

Besonders bevorzugt ist das erfindungsgemäße Verfahren dadurch gekennzeichnet, daß die Konzentration des chaotropen Hilfsstoffs in Schritt (b) etwa 8 M, die Temperatur in Schritt (b) etwa 40 °C, der pH-Wert in Schritt (b) etwa 10,2, der pH-Wert in Schritt (c) etwa 10,6 beträgt und in Schritt (c) die Menge an Wasser eine Verdünnung der Konzentration des Cysteins oder des Cysteinhydrochlorids in dem Gemisch auf etwa 2,5 bis 3,0 mM und eine Konzentration des chaotropen Hilfsstoffs von 0,5 M ergibt.

Das Ergebnis des Verfahrens gemäß der vorliegenden Erfindung ist ein Vorläufer von Insulinen oder Insulinderivaten, im besonderen ein Proinsulin, dessen Cysteinbrücken korrekt verbunden sind.

Insulinderivate sind Derivate von natürlich vorkommenden Insulinen, nämlich Humaninsulin (s. SEQ ID NO 1 = A-Kette von Humaninsulin; s. SEQ ID NO 2 = B-Kette von Humaninsulin, Sequenzprotokoll) oder tierischen Insulinen, welche sich durch Substitution wenigstens eines natürlich auftretenden Aminosäurerestes und/oder Addition wenigstens eines Aminosäurerestes und/oder organischen Restes von dem entsprechenden, ansonst gleichen natürlich vorkommenden Insulin unterscheiden.

Aus dem mit Hilfe des Verfahrens gemäß der vorliegenden Erfindung gewonnenen Vorläufer der Insuline bzw. Insulinderivate mit korrekt verbundenen Cystinbrücken kann schließlich nach dem in der EP 0 600 372 A1 (bzw. US 5,473,049) oder in der EP 0 668 292 A2 beschriebenen Verfahren durch enzymatische Spaltung mittels Trypsin oder eines trypsinähnlichen Enzyms und gegebenenfalls zusätzlich mittels Carboxypeptidase B und anschließender Reinigung an einem Adsorberharz ein Insulin oder ein Insulinderivat mit korrekt verbundenen Cystinbrücken hergestellt werden.

Das aus dem Vorläufer herstellbare Insulin oder Insulinderivat läßt sich vorzugsweise durch Formel I beschreiben 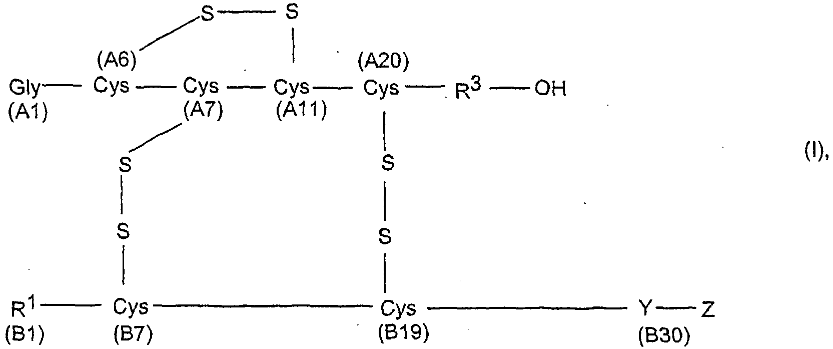 worin bedeuten
- Y: ein genetisch kodierbarer Aminosäurerest,
- Z: a) ein Aminosäurerest aus der Gruppe His, Arg oder Lys,
b) ein Peptid mit 2 oder 3 Aminosäureresten, enthaltend den Aminosäurerest Arg oder Lys am Carboxylende des Peptids,
c) ein Peptid mit 2 - 35 genetisch kodierbaren Aminosäuren, enthaltend 1 bis 5 Histidinreste, oder
d) OH,
- R¹: ein Phenylalaninrest (Phe) oder eine kovalente Bindung,
- R³: ein genetisch kodierbarer Aminosäurerest,
wobei die zur Vereinfachung der Formel I nicht gezeigten Reste A2 - A20 der Aminosäuresequenz der A-Kette von Humaninsulin, tierischen Insulin oder einem Insulinderivat und die zur Vereinfachung der Formel I nicht gezeigten Reste B2 - B29 der Aminosäuresequenz der B-Kette von Humaninsulins, tierischen Insulin oder einem Insulinderivat entsprechen.

Die Aminosäuresequenz von Peptiden und Proteinen wird vom N-terminalen Ende der Aminosäurekette an bezeichnet. Die in Formel I in Klammern gemachten Angaben, z.B. A6, A20, B1, B7 oder B19, entsprechen der Position von Aminosäureresten in den A- oder B-Ketten des Insulins.

Für den Begriff "genetisch kodierbarer Aminosäurerest" stehen die Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.

Für die Begriffe "Reste A2 - A20" und "Reste B2 - B 29" von tierischem Insulin werden beispielsweise die Aminosäuresequenzen von Insulin aus Rindern, Schweinen oder Hühnern verstanden. Der Begriff "Reste A2 - A20" und "B2 - B29" von Insulinderivaten steht für die entsprechenden Aminosäuresequenzen von Humaninsulin, die durch den Austausch von Aminosäuren durch andere genetisch kodierbare Aminosäuren gebildet werden.

Die A-Kette von Humaninsulin hat beispielsweise folgende Sequenz (SEQ ID NO.: 1):

Gly Ile Val Glu Gln Cys Cys Thr Ser Ile Cys Ser Leu Tyr Gln Leu
Glu Asn Tyr Cys Asn.

Die B-Kette von Humaninsulin hat folgende Sequenz (SEQ ID NO.: 2): 
Phe Val Asn Gln His Leu Cys Gly Ser His Leu Val Glu Ala Leu Tyr
Leu Val Cys Gly Glu Arg Gly Phe Phe Tyr Thr Pro Lys Thr.
 Dabei ist in Formel I R³ Asparagin (Asn), R¹ Phenylalanin (Phe), Y Threonin (Thr) und Z OH.

Das Verfahren gemäß der vorliegenden Erfindung ist dementsprechend besonders zur Gewinnung eines Vorläufers von Insulinen oder Insulinderivaten mit der allgemeinen Formel II, dessen Cystinbrücken (in Formel II nicht gezeigt) korrekt gefaltet sind,

R²-R¹-(B2-B29)-Y-X-Gly-(A2-A20)-R³ (II)

geeignet,
worin bedeuten
- R²: a) ein Wasserstoffatom,
b) ein Aminosäurerest aus der Gruppe Lysin (Lys) oder Arginin (Arg) oder
c) ein Peptid mit 2 bis 45 Aminosäureresten, enthaltend den Aminosäurerest Lysin (Lys) oder Arginin (Arg) am Carboxylende des Peptids
- R¹: ein Phenylalaninrest (Phe) oder eine kovalente Bindung,
- (B2-B29): die Aminosäurereste in den Positionen B2 bis B29 der B-Kette von Humaninsulin, tierischem Insulin oder einem gegebenenfalls in einer oder mehrerer dieser Positionen variierten Insulinderivat,
- Y: ein genetisch kodierbarer Aminosäurerest,
- X: a) ein Aminosäurerest aus der Gruppe Lysin (Lys) oder Arginin (Arg),
b) ein Peptid mit 2 bis 35 Aminosäureresten, enthaltend den Aminosäurerest Lysin (Lys) oder Arginin (Arg) am N-terminalen Ende und amCarboxylende des Peptids, oder
c) ein Peptid mit 2 bis 35 genetisch kodierbaren Aminosäuren, enthaltend 1 bis 5 Histidinreste,
- (A2-A20): die Aminosäurereste in den Positionen A2 bis A20 der B-Kette von Humaninsulin, tierischem Insulin oder einem gegebenenfalls in einer oder mehrerer dieser Positionen variierten Insulinderivat und
- R³: ein genetisch kodierbarer Aminosäurerest.

### 1. Vorzugsweise bedeuten in Formel II:

- R²: a) ein Wasserstoffatom oder
b) ein Peptid mit 2 bis 25 Aminosäureresten, enthaltend den Aminosäurerest Arginin (Arg) am Carboxylende des Peptids,
- R¹: ein Phenylalaninrest (Phe),
- (B2-B29): die Aminosäurereste in den Positionen B2 bis B29 der B-Kette von Humaninsulin,
- Y: ein Aminosäurerest aus der Gruppe Alanin (Ala), Threonin (Thr) oder Serin (Ser),
- X: der Aminosäurerest Arginin (Arg) oder ein Peptid mit der Aminosäuresequenz der C-Kette von Humaninsulin,
- (A2-A20): die Aminosäurereste in den Positionen A2 bis A20 der B-Kette von Humaninsulin und
- R³: ein Aminosäurerest aus der Gruppe Asparagin (Asn), Serin (Ser) oder Glycin (Gly).

Die C-Kette von Humaninsulin hat folgende Sequenz (SEQ ID NO.: 3):

Arg Arg Glu Ala Glu Asp Leu Gln Val Gly Gln Val Glu Leu Gly Gly
Gly Pro Gly Ala Gly Ser Leu Gln Pro Leu Ala Leu Glu Gly Ser Leu
Gln Lys Arg.

### 2. Vorzugsweise bedeuten in Formel II:

- R²: a) ein Wasserstoffatom oder
b) ein Peptid mit 2 bis 15 Aminosäureresten, an dessen Carboxylende sich ein Argininrest (Arg) befindet,
- R¹: ein Phenylalaninrest (Phe),
- (B2-B29): die Aminosäurereste in den Positionen B2 bis B29 der B-Kette von Humaninsulin,
- Y: ein Threoninrest (Thr),
- X: der Aminosäurerest Arginin (Arg) oder ein Peptid mit 2 bis 35 Aminosäureresten, wobei am Anfang und am Ende des Peptids zwei basische Aminosäurereste, insbesondere Arginin (Arg) und/oder Lysin (Lys) stehen,
- (A2-A20): die Aminosäurereste in den Positionen A2 bis A20 der B-Kette von Humaninsulin und
- R³: der Aminosäurerest Asparagin (Asn) oder Glycin (Gly).

Der Rest Z des Insulins oder des Insulinderivates der Formel I ist in der Regel Teil der Aminosäuresequenz von X des Vorläufers der Formel II und entsteht durch die Aktivität der Proteasen wie Trypsin, trypsinähnliches Enzym oder Carboxypeptidase B. Der Rest R³ ist der Aminosäurerest, der an Position A21 der A-Kette von Insulin steht. Der Rest Y ist der Aminosäurerest, der an Position B30 der B-Kette von Insulin steht.

Trypsin oder trypsinähnliche Enzyme sind Proteasen, die Aminosäureketten am Arginin- oder Lysinrest spalten.

Carboxypeptidase B ist eine Exoprotease, die basische Aminosäurereste wie Arg oder Lys, die am Carboxyterminalen Ende von Aminosäureketten stehen, abspaltet. (Kemmler et al., J. Biol. Chem. 246, Seiten 6786-6791).

Aus dem unter 1 genannten Vorläufer läßt sich beispielsweise ein Insulin bzw. Insulinderivat der Formel I mit korrekt verknüpften Cystinbrücken gewinnen, wobei Y, R¹, R², R³, A2-A20 und B2-B29 die unter 1 genannte Bedeutung haben und Z ein Argininrest (Arg), ein Peptidrest Arg-Arg oder -OH bedeutet.

Aus dem unter 2 genannten Vorläufer läßt sich beispielsweise ein Insulin bzw. Insulinderivat der Formel I mit korrekt verknüpften Cystinbrücken gewinnen, wobei Y, R¹, R², R³, A2-A20 und B2-B29 die unter 2 genannte Bedeutung haben und Z ein Argininrest (Arg), ein Peptidrest Arg-Arg oder Lys-Lys oder -OH bedeutet.

Der Vorläufer der Formel II kann mit Hilfe einer Vielzahl gentechnischer Konstrukte in Mikroorganismen gebildet werden (EP 0 489 780, EP 0 347 781, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Für das erfindungsgemäße Verfahren können Vorläufer von Insulinen oder von Insulinderivaten der Formel II eingesetzt werden, die direkt nach dem Zellaufschluß noch mit einer Vielzahl von Proteinen, die aus der Fermentationslösung und aus den Mikroorganismen stammen, verunreinigt sind. Die Vorläufer der Formel II können aber auch in vorgereinigter Form beispielsweise nach einer Fällung oder einer chromatographischen Reinigung eingesetzt werden.

Die Erfindung wird im folgenden durch Beispiele erläutert, ohne sich darauf zu beschränken.

### Beispiel 1 (Vergleichsbeispiel, Stand der Technik)

Durch Fermentation von genetisch modifizierten Escherichia-coli-Zellen (EP 0 489 780) wird ein Fusionsprotein mit folgender Aminosäuresequenz hergestellt. Proinsulinsequenz 1 (Seq Id No. 4):

Ala Thr Thr Ser Thr Gly Asn Ser Ala Arg Phe Val Asn Gln His
Leu Cys Gly Ser His Leu Val Glu Ala Leu Tyr Leu Val Cys Gly Glu
Arg Gly Phe Phe Tyr Thr Pro Lys Thr Arg Arg Glu Ala Glu Asp Leu
Gln Val Gly Gln Val Glu Leu Gly Gly Gly Pro Gly Ala Gly Ser Leu
Gln Pro Leu Ala Leu Glu Gly Ser Leu Gln Lys Arg Gly Ile Val Glu
Gln Cys Cys Thr Ser Ile Cys Ser Leu Tyr Gln Leu Glu Asn Tyr Cys
Asn

Proinsulinsequenz 1 entspricht der Formel II, dabei ist
- X: C-Peptid von Humaninsulin,
- Y: Thr (B30),
- R¹: Phe (B1),
- R²: ein Peptid mit 11 Aminosäureresten,
- R³: Gly (A21) und
A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

In den E.-coli-Zellen sammelt sich das exprimierte Fusionsprotein mit der Proinsulinsequenz 1 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörperchen werden durch Zentrifugation isoliert.

Der wässrigen Fusionsproteinsuspension, die 40 kg Fusionsprotein (Ermittelt durch Gefriertrocknung eines Aliquots) enthalten, werden 5 kg Cysteinhydrochloridhydrat zugefügt.

Die Suspension wird (Der Anteil des insulinhaltigen Fusionsproteins wird mit Hilfe der HPLC bestimmt. Er beträgt 50 %.) mit der Proinsulinsequenz 1 in 550 I einer 8 M Harnstoff-Lösung bei pH 10,2 bei 40°C gelöst. Die klare Lösung wird in 9000 I Wasser bei einem pH-Wert von 10,6 und einer Temperatur von 16° C eingerührt. Nach 4 Std. unter Rühren wird mit Hilfe der analytischen HPLC der Gehalt an Proinsulinsequenz I mit korrekt verbundenen Cystinbrücken im Reaktionsansatz mit 5,0 kg entsprechend einem Umsatz von 25 % bestimmt.

Die 9500 I Lösung wird mit 1 N HCl auf einen pH von 5,0 eingestellt und separiert. Dann erfolgt durch Zugabe von 1 N Natronlauge die Einstellung von pH 9. Es werden 10 g Trypsin in die Lösung eingetragen. Es entsteht ca. 2,2 kg Insulinvorläufer 2 nach HPLC-Messung.

Insulin 2 entspricht der Formel I, dabei ist
- Y: Thr (B30),
- Z: Arg-Arg,
- R¹: Phe (B1),
- R³: Gly (A21) und

A2 - A20 die Aminosäuresequenz der A-Kette von Humaninsulin (Aminosäurereste 2 bis 20) und B2-B29 die Aminosäuresequenz der B-Kette von Humaninsulin (Aminosäurereste 2 bis 29).

### Insulin 2 besteht aus einer A-Kette mit der Sequenz

Gly Ile Val Glu Gln Cys Cys Thr Ser Ile Cys Ser Leu Tyr Gln Leu Glu Asn Tyr Cys Gly (SEQ ID NO: 5)
und einer B-Kette der Sequenz 
Phe Val Asn Gln His Leu Cys Gly Ser His Leu Val Glu Ala Leu Tyr Leu Val Cys Gly
Glu Arg Gly Phe Phe Tyr Thr Pro Lys Thr Arg Arg (SEQ ID NO: 6)
 die über korrekt gebundene Cystinbrücken miteinander verbunden sind.

Die Lösung wird mittels Adsorberharz aufkonzentriert und gereinigt.

Das Eluat, das Insulin 2 enthält, kann nach Verdünnung mit Wasser und pH-Einstellung unmittelbar auf einer Chromatographiesäule weiter gereinigt werden.

### Beispiel 2 (Verfahren gemäß der vorliegenden Erfindung)

Durch Fermentation von genetisch modifizierten Escherichia-coli-Zellen (EP 0 489 780) wird ein Fusionsprotein mit der Aminosäuresequenz Proinsulinsequenz 1 (SEQ ID NO: 4) hergestellt.

In den E.-coli-Zellen sammelt sich das exprimierte Fusionsprotein mit der Proinsulinsequenz 1 an und bildet Einschlusskörper. Nach Beendigung der Fermentation werden die Zellen durch Zentrifugation abgetrennt und durch übliche Hochdruckhomogenisation aufgeschlossen. Die freigesetzten Fusionsproteineinschlusskörper werden durch Zentrifugation isoliert.

Der wässrigen Fusionsproteinsuspension, die 40 kg Fusionsprotein (Ermittelt durch Gefriertrocknung eines Aliquots) enthalten, werden 5 kg Cysteinhydrochloridhydrat zugefügt.

Die Suspension wird (Der Anteil des insulinhaltigen Fusionsproteins wird mit Hilfe der HPLC bestimmt. Er beträgt 50 %.) mit der Proinsulinsequenz 1 in 550 I einer 8 M Harnstoff-Lösung bei pH 10,2 bei 40°C gelöst. Die klare Lösung wird in 9000 I Wasser bei einem pH-Wert von 10,6 und einer Temperatur von 15° C eingerührt. Der Gasraum des Behälters wird dabei mit einer Streichluftmenge von 4m³/h während der gesamten Reaktionszeit unter Rühren des Ansatzes begast. In dem 10000 I fassenden Behälter von 2000 mm Durchmesser und zwei Stromstörern über den zylindrischen Bereich werden die 9500I Faltlösung von einem dreistufigen Trapezrührer mit einem Rührorgandurchmesser von 1100 mm und einer elektrischen Antriebsleistung von 2 kW derart umgewälzt, dass eine bodengängige Gasdurchmischung des Inhalts gewährleistet ist. Das Volumen/Oberfläche-Verhältnis des Reaktionsansatzes beträgt 1:3,14. Der Sauerstoffgehalt wurde durch Rühren und Begasen bei 8 mg/l gehalten.

Nach Abschluss der Faltungsreaktion nach 4 Std. wird mit Hilfe der analytischen HPLC der Gehalt an Proinsulinsequenz I mit korrekt verbundenen Cystinbrücken im Reaktionsansatz mit 10,0 kg entsprechend einem Umsatz von 50 % bestimmt.

Die 9500 I Lösung wird mit 1 N HCl auf einen pH von 5,0 eingestellt und separiert. Dann erfolgt durch Zugabe von 1 N Natronlauge die Einstellung von pH 9. Es werden 10 g Trypsin in die Lösung eingetragen. Es entsteht 4,5 kg Insulin 2 nach HPLC-Messung.

Die Lösung wird mittels Adsorberharz aufkonzentriert und gereinigt.

Das Eluat, das Insulin 2 enthält, kann nach Verdünnung mit Wasser und pH-Einstellung unmittelbar auf einer Chromatographiesäule weiter gereinigt werden.

## Patentansprüche

1. Verfahren zur Herstellung von Insulinen, oder von einem Insulinderivat mit korrekt verbundenen Cysteinbrücken aus einem Vorläufer des Insulin oder des Insulinderivats, wobei der Vorläufer in Gegenwart von Cystein oder Cysteinhydrochlorid und von einem chaotropen Hilfsstoff einem Faltungsprozess unterworfen wird, wonach aus dem Vorläufer durch enzymatische Spaltung mittels Trypsin oder eines trypsinähnlichen Enzyms und gegebenenfalls zusätzlich mittels Carboxypeptidase B und anschließender Reinigung an einem Adsorberharz ein Insulin oder ein Insulinderivat mit korrekt verbundenen Cysteinbrücken gewonnen wird, **dadurch gekennzeichnet, dass** man zur Ausführung des Faltungsprozesses nacheinander die folgenden Schritte durchführt:
(a) Versetzen einer wässrigen Suspension des Vorläufers von Insulinen oder Insulinderivaten mit einer Menge an Cystein oder Cysteinhydrochlorid, die 1 bis 15 SH-Reste des Cysteins oder Cysteinhydrochlorids pro Cysteinrest des Vorläufers ergibt,
(b) Eintragen der cystein- oder cysteinhydrochloridhaltigen Suspension des Vorläufers in eine 4 bis 9 molare Lösung des chaotropen Hilfsstoffs bei einem pH-Wert von 8 bis 11,5 und einer Temperatur von ungefähr 15 bis 55 °C, Halten des erhaltenen Gemischs für etwa 10 bis 60 Minuten auf dieser Temperatur und
(c) Eintragen des Gemischs bei einem pH-Wert von 8 bis 11,5 und einer Temperatur von 5 bis 30 °C in eine Menge an Wasser, welche eine Verdünnung der Konzentration des Cysteins oder des Cysteinhydrochlorids in dem Gemisch auf etwa 1 bis 5 mM und des chaotropen Hilfsstoffs auf 0,2 bis 1,0 M ergibt,
wobei das Gemisch in Schritt (c) in einem Behälter begast wird, so dass die Sauerstoffkonzentration in der Suspension 1 bis 15 mg/l ist.

2. Verfahren nach Anspruch 1, wobei das Verhältnis von Volumen zu Oberfläche des Gemisches in Schritt (c) größer als 1 ist.

3. Verfahren nach Anspruch 2, wobei das Verhältnis von Volumen zu Oberfläche des Gemisches größer als 2 ist.

4. Verfahren nach einem oder mehreren der Ansprüche 1 bis 3, wobei die Sauerstoffkonzentration in dem Gemisch 2 bis 10 mg/l beträgt.

5. Verfahren nach einem oder mehreren der Ansprüche 1 bis 4, **dadurch gekennzeichnet, dass** in Schritt (a) die Menge an Cystein oder Cysteinhydrochlorid einer Menge entspricht, die 1 bis 6 SH-Reste des Cysteins oder Cysteinhydrochlorids pro Cysteinrest des Vorläufers ergibt,
in Schritt (b) das Eintragen der cystein- oder cysteinhydrochloridhaltigen Suspension des Vorläufers in eine 4 bis 9 molare Lösung des chaotropen Hilfsstoffs bei einem pH-Wert von 8 bis 11 und einer Temperatur von 30 bis 45 °C, das Halten des erhaltenen Gemischs für 20 bis 40 Minuten auf dieser Temperatur und
in Schritt (c) das Eintragen des Gemischs bei einem pH-Wert von 8 bis 11 und bei einer Temperatur von 15 bis 20 °C in eine Menge an Wasser, welche eine Verdünnung der Konzentration des Cysteins oder des Cysteinhydrochlorids in dem Gemisch auf etwa 1 bis 5 mM ergibt und eine Konzentration des chaotropen Hilfsstoffs von 0,2 bis 1,0 M, erfolgt.

6. Verfahren nach einem oder mehreren der Ansprüche 1 bis 5, **dadurch gekennzeichnet, daß** der chaotrope Hilfsstoff Guanidin, Guanidinhydrochlorid oder Harnstoff ist.

7. Verfahren nach einem oder mehreren der Ansprüche 1 bis 6, **dadurch gekennzeichnet, daß** die Konzentration des chaotropen Hilfsstoffs in Schritt (b) 7,0 bis 9 M beträgt.

8. Verfahren nach einem oder mehreren der Ansprüche 1 bis 7, **dadurch gekennzeichnet, dass** die Temperatur in Schritt (b) 40 °C beträgt.

9. Verfahren nach einem oder mehreren der Ansprüche 1 bis 8, **dadurch gekennzeichnet, dass** der pH-Wert in Schritt (b) 10 bis 11 beträgt.

10. Verfahren nach einem oder mehreren der Ansprüche 1 bis 9, **dadurch gekennzeichnet, dass** der pH-Wert in Schritt (c) 10 bis 11 beträgt.

11. Verfahren nach einem oder mehreren der Ansprüche 1 bis 10, **dadurch gekennzeichnet, dass** in Schritt (c) die Menge an Wasser eine Verdünnung der Konzentration des Cysteins oder des Cysteinhydrochlorids in dem Gemisch auf 2,5 bis 3,0 mM und eine Konzentration des chaotropen Hilfsstoffs auf 0,5 M ergibt.

12. Verfahren nach einem oder mehreren der Ansprüche 1 bis 11, **dadurch gekennzeichnet, dass** die Konzentration des chaotropen Hilfsstoffs in Schritt (b) etwa 8 M, die Temperatur in Schritt (b) etwa 40 °C, der pH-Wert in Schritt (b) etwa 10,2, der pH-Wert in Schritt (c) etwa 10,6 beträgt und in Schritt (c) die Menge an Wasser eine Verdünnung der Konzentration des Cysteins oder des Cysteinhydrochlorids in dem Gemisch auf etwa 2,5 bis 3,0 mM und eine Konzentration des chaotropen Hilfsstoffs von 0,5 M ergibt.

13. Verfahren nach einem oder mehreren der Ansprüche 1 bis 12, **dadurch gekennzeichnet, dass** der Vorläufer der Insuline oder der Insulinderivate die Sequenz gemäß der allgemeinen Formel II aufweist
R²-R¹-(B2-B29)-Y-X-Gly-(A2-A20)-R³ (II),
worin bedeuten
R² a) ein Wasserstoffatom,
b) ein Aminosäurerest aus der Gruppe Lysin (Lys) oder Arginin (Arg) oder
c) ein Peptid mit 2 bis 45 Aminosäureresten, enthaltend den Aminosäurerest Lysin (Lys) oder Arginin (Arg) am Carboxylende des Peptids
R¹ ein Phenylalaninrest (Phe) oder eine kovalente Bindung,
(B2-B29) die Aminosäurereste in den Positionen B2 bis B29 der B-Kette von Humaninsulin, tierischem Insulin oder einem gegebenenfalls in einer oder mehrerer dieser Positionen variierten Insulinderivat,
Y ein genetisch kodierbarer Aminosäurerest,
X a) ein Aminosäurerest aus der Gruppe Histin (His), Lysin (Lys) oder Arginin (Arg) oder
b) ein Peptid mit 2 bis 35 Aminosäureresten, enthaltend den Aminosäurerest Lysin (Lys) oder Arginin (Arg) am N-terminalen Ende und amCarboxylende des Peptids, oder
c) ein Peptid mit 2 bis 35 genetisch kodierbaren Aminosäuren, enthalend 1 bis 5 Histidinreste,
(A2-A20) die Aminosäurereste in den Positionen A2 bis A20 der B-Kette von Humaninsulin, tierischem Insulin oder einem gegebenenfalls in einer oder mehrerer dieser Positionen variierten Insulinderivat und
R³ ein genetisch kodierbarer Aminosäurerest.

14. Verfahren nach Anspruch 13, **dadurch gekennzeichnet, daß** in Formel II
R² a) ein Wasserstoffatom oder
b) ein Peptid mit 2 bis 25 Aminosäureresten, enthaltend den Aminosäurerest Arginin (Arg) am Carboxylende des Peptids,
R¹ ein Phenylalaninrest (Phe),
(B2-B29) die Aminosäurereste in den Positionen B2 bis B29 der B-Kette von Humaninsulin,
Y ein Aminosäurerest aus der Gruppe Alanin (Ala), Threonin (Thr) oder Serin (Ser),
X der Aminosäurerest Arginin (Arg) oder ein Peptid mit der Aminosäuresequenz der C-Kette von Humaninsulin,
(A2-A20) die Aminosäurereste in den Positionen A2 bis A20 der B-Kette von Humaninsulin und
R³ ein Aminosäürerest aus der Gruppe Asparagin (Asn), Serin (Ser) oder Glycin (Gly) bedeuten.

15. Verfahren nach Anspruch 13, **dadurch gekennzeichnet, daß** in Formel II
R² a) ein Wasserstoffatom oder
b) ein Peptid mit 2 bis 15 Aminosäureresten, an dessen Carboxylende sich ein Argininrest (Arg) befindet,
R¹ ein Phenylalaninrest (Phe),
(B2-B29) die Aminosäurereste in den Positionen B2 bis B29 der B-Kette von Humaninsulin,
Y ein Threoninrest (Thr),
X der Aminosäurerest Arginin (Arg) oder ein Peptid mit 2 bis 35 Aminosäureresten, wobei am Anfang und am Ende des Peptids zwei basische Aminosäurereste, insbesondere Arginin (Arg) und/oder Lysin (Lys) stehen,
(A2-A20) die Aminosäurereste in den Positionen A2 bis A20 der B-Kette von Humaninsulin und
R³ der Aminosäurerest Asparagin (Asn) oder Glycin (Gly)
bedeuten.

## Claims

1. A process for preparing insulins or an insulin derivative with correctly linked cysteine bridges from a precursor of said insulin or insulin derivative, wherein said precursor is subjected to a folding process in the presence of cysteine or cysteine hydrochloride and of a chaotropic auxiliary compound, whereafter an insulin or insulin derivative with correctly linked cysteine bridges is obtained by enzymic cleavage by means of trypsin or a trypsin-like enzyme and, where appropriate, additionally by means of carboxypeptidase B and subsequent purification on an adsorber resin, which process comprises carrying out said folding process by performing sequentially the following steps:
(a) adding an amount of cysteine or cysteine hydrochloride to an aqueous suspension of the precursor of insulins or insulin derivatives, resulting in 1 to 15 SH radicals of said cysteine or cysteine hydrochloride per cysteine residue of said precursor,
(b) introducing the cysteine- or cysteine hydrochloride-containing suspension of the precursor into a 4 to 9 molar solution of the chaotropic auxiliary compound at a pH of from 8 to 11.5 and a temperature of from 15 to 55 °C, keeping the resulting mixture at this temperature for about 10 to 60 minutes, and
(c) introducing said mixture at a pH of from 8 to 11.5 and a temperature of from 5 to 30 °C into an amount of water resulting in the concentration of said cysteine or cysteine hydrochloride in said mixture being diluted to from about 1 to 5 mM and that of said chaotropic auxiliary compound being diluted to from 0.2 to 1.0 M,
the mixture in step (c) being gassed in a container so that the concentration of oxygen in the suspension is from 1 to 15 mg/l.

2. The process as claimed in claim 1, wherein the volume-to-surface ratio of the mixture in step (c) is greater than 1.

3. The process as claimed in claim 2, wherein the volume-to-surface ratio of the mixture is greater than 2.

4. The process as claimed in one or more of claims 1 to 3, wherein the concentration of oxygen in the mixture is from 2 to 10 mg/l.

5. The process as claimed in one or more of claims 1 to 4, wherein, in step (a), the amount of cysteine or cysteine hydrochloride corresponds to an amount resulting in from 1 to 6 SH radicals of said cysteine or cysteine hydrochloride per cysteine residue of the precursor,
in step (b), the cysteine- or cysteine hydrochloride-containing suspension of the precursor is introduced into a 4 to 9 molar solution of the chaotropic auxiliary compound at a pH of from 8 to 11 and a temperature of from 30 to 45 °C, the resulting mixture is kept at this temperature for 20 to 40 minutes, and,
in step (c), said mixture is introduced at a pH of from 8 to 11 and a temperature of from 15 to 20 °C into an amount of water resulting in the concentration of said cysteine or cysteine hydrochloride in said mixture being diluted to from about 1 to 5 mM and in a concentration of said chaotropic auxiliary compound of from 0.2 to 1.0 M.

6. The process as claimed in one or more of claims 1 to 5, wherein the chaotropic auxiliary compound is guanidine, guanidine hydrochloride or urea.

7. The process as claimed in one or more of claims 1 to 6, wherein the concentration of the chaotropic auxiliary compound in step (b) is from 7.0 to 9 M.

8. The process as claimed in one or more of claims 1 to 7, wherein the temperature in step (b) is 40 °C.

9. The process as claimed in one or more of claims 1 to 8, wherein the pH in step (b) is from 10 to 11.

10. The process as claimed in one or more of claims 1 to 9, wherein the pH in step (c) is from 10 to 11.

11. The process as claimed in one or more of claims 1 to 10, wherein, in step (c), the amount of water results in the concentration of the cysteine or cysteine hydrochloride in the mixture being diluted to from 2.5 to 3.0 mM and in a concentration of the chaotropic auxiliary compound of 0.5 M.

12. The process as claimed in one or more of claims 1 to 11, wherein the concentration of the chaotropic auxiliary compound in step (b) is about 8 M, the temperature in step (b) is about 40 °C, the pH in step (b) is about 10.2, the pH in step (c) is about 10.6 and, in step (c), the amount of water results in the concentration of the cysteine or cysteine hydrochloride in the mixture being diluted to from about 2.5 to 3.0 mM and in a concentration of the chaotropic auxiliary compound of 0.5 M.

13. The process as claimed in one or more of claims 1 to 12, wherein the precursor of the insulins or insulin derivatives has the sequence of the general formula II
R²-R¹-(B2-B29)-Y-X-Gly-(A2-A20)-R³ (II),
where
R² a) is hydrogen,
b) is an amino acid residue from the group consisting of lysine (Lys) and arginine (Arg), or
c) is a peptide having from 2 to 45 amino acid residues, comprising the amino acid residue lysine (Lys) or arginine (Arg) at the carboxyl end of said peptide,
R¹ is a phenylalanine residue (Phe) or a covalent bond,
(B2-B29) are the amino acid residues in positions B2 to B29 of the B chain of human insulin, animal insulin or of an insulin derivative which may have been modified in one or more of said positions,
Y is a genetically encodable amino acid residue,
X a) is an amino acid residue from the group consisting of histine (His), lysine (Lys) and arginine (Arg), or
b) is a peptide having from 2 to 35 amino acid residues, comprising the amino acid residue lysine (Lys) or arginine (Arg) at the N terminus and at the carboxyl end of said peptide, or
c) is a peptide having from 2 to 35 genetically encodable amino acids, comprising from 1 to 5 histidine residues,
(A2-A20) are the amino acid residues in positions A2 to A20 of the B chain of human insulin, animal insulin or of an insulin derivative which may have been modified in one or more of said positions, and
R³ is a genetically encodable amino acid residue.

14. The process as claimed in claim 13, wherein, in the formula II,
R² a) is hydrogen, or
b) is a peptide having from 2 to 25 amino acid residues, comprising the amino acid residue arginine (Arg) at the carboxyl end of said peptide,
R¹ is a phenylalanine residue (Phe),
(B2-B29) are the amino acid residues in positions B2 to B29 of the B chain of human insulin,
Y is an amino acid residue from the group consisting of alanine (Ala), threonine (Thr) and serine (Ser),
X is the amino acid residue arginine (Arg) or a peptide having the amino acid sequence of the C chain of human insulin,
(A2-A20) are the amino acid residues in positions A2 to A20 of the B chain of human insulin, and
R³ is an amino acid residue from the group consisting of asparagine (Asn), serine (Ser) and glycine (Gly).

15. The process as claimed in claim 13, wherein, in the formula II,
R² a) is hydrogen, or
b) is a peptide having from 2 to 15 amino acid residues, whose carboxyl end comprises an arginine residue (Arg),
R¹ is a phenylalanine residue (Phe),
(B2-B29) are the amino acid residues in positions B2 to B29 of the B chain of human insulin,
Y is a threonine residue (Thr),
X is the amino acid residue arginine (Arg) or a peptide having from 2 to 35 amino acid residues, whose start and end comprise two basic amino acid residues, in particular arginine (Arg) and/or lysine (Lys),
(A2-A20) are the amino acid residues in positions A2 to A20 of the B chain of human insulin, and
R³ is the amino acid residue asparagine (Asn) or glycine (Gly).

## Revendications

1. Procédé de préparation d'insulines ou d'un dérivé de l'insuline comportant des ponts cystéine correctement reliés, à partir d'un précurseur de l'insuline ou du dérivé de l'insuline, le précurseur étant soumis à un processus de repliement en présence de cystéine ou de chlorhydrate de cystéine et d'un adjuvant chaotropique, ce après quoi on obtient, à partir du précurseur, par clivage enzymatique à l'aide de trypsine ou d'une enzyme analogue à la trypsine, et éventuellement en outre à l'aide de carboxypeptidase B, suivi d'une purification sur une résine adsorbante, une insuline ou un dérivé de l'insuline comportant des ponts cystéine correctement reliés, **caractérisé en ce que**, pour exécuter le processus de repliement, on met en oeuvre successivement les étapes suivantes :
(a) addition, à une suspension aqueuse du précurseur d'insuline ou de dérivés de l'insuline, d'une quantité de cystéine ou de chlorhydrate de cystéine, qui donne 1 à 15 résidus SH de la cystéine ou du chlorhydrate de cystéine par résidu cystéine du précurseur,
(b) introduction de la suspension du précurseur, contenant de la cystéine ou du chlorhydrate de cystéine, dans une solution 4 à 9 molaire de l'adjuvant chaotropique, à un pH de 8 à 11,5 et à une température d'environ 15 à 55°C, maintien du mélange obtenu pendant environ 10 à 60 minutes à cette température, et
(c) introduction du mélange, à un pH de 8 à 11,5 et à une température de 5 à 30°C, dans une quantité d'eau qui donne une dilution de la concentration de la cystéine ou du chlorhydrate de cystéine dans le mélange à environ 1 à 5 mM, et de l'adjuvant chaotropique à 0,2 à 1,0 M,
le mélange dans l'étape (c) étant purgé dans un récipient de façon que la concentration de l'oxygène dans la suspension soit de 1 à 15 mg/l.

2. Procédé selon la revendication 1, dans lequel le rapport du volume à l'aire du mélange dans l'étape (c) est supérieur à 1.

3. Procédé selon la revendication 2, dans lequel le rapport du volume à l'aire du mélange est supérieur à 2.

4. Procédé selon l'une ou plusieurs des revendications 1 à 3, dans lequel la concentration de l'oxygène dans le mélange est de 2 à 10 mg/l.

5. Procédé selon l'une ou plusieurs des revendications 1 à 4, **caractérisé en ce que** dans l'étape (a) la quantité de cystéine ou de chlorhydrate de cystéine correspond à une quantité donnant 1 à 6 résidus SH de la cystéine ou du chlorhydrate de cystéine par résidu de cystéine du précurseur,
dans l'étape (b) l'introduction de la suspension du précurseur contenant de la cystéine ou du chlorhydrate de cystéine dans une solution 4 à 9 molaire de l'adjuvant chaotropique est réalisée à un pH de 8 à 11 et à une température de 30 à 45°C, le maintien du mélange obtenu est réalisé pendant 20 à 40 minutes à cette température, et
dans l'étape (c), l'introduction du mélange est réalisée à un pH de 8 à 11 et à une température de 15 à 20°C dans une quantité d'eau qui donne une dilution de la concentration de la cystéine ou du chlorhydrate du cystéine dans un mélange d'environ 1 à 5 mM, et à une concentration de l'adjuvant chaotropique de 0,2 à 1,0 M.

6. Procédé selon l'une ou plusieurs des revendications 1 à 5, **caractérisé en ce que** l'adjuvant chaotropique est la guanidine, le chlorhydrate de guanidinium ou l'urée.

7. Procédé selon l'une ou plusieurs des revendications 1 à 6, **caractérisé en ce que** la concentration de l'adjuvant chaotropique dans l'étape (b) est de 7,0 à 9 M.

8. Procédé selon l'une ou plusieurs des revendications 1 à 7, **caractérisé en ce que** la température dans l'étape (b) est de 40°C.

9. Procédé selon l'une ou plusieurs des revendications 1 à 8, **caractérisé en ce que** le pH dans l'étape (b) est de 10 à 11.

10. Procédé selon l'une ou plusieurs des revendications 1 à 9, **caractérisé en ce que** le pH dans l'étape (c) est de 10 à 11.

11. Procédé selon l'une ou plusieurs des revendications 1 à 10, **caractérisé en ce que** dans l'étape (c) la quantité d'eau donne une dilution de la concentration de la cystéine ou du chlorhydrate de cystéine dans le mélange de 2,5 à 3,0 mM et une concentration de l'adjuvant chaotropique de 0,5 M.

12. Procédé selon l'une ou plusieurs des revendications 1 à 11, **caractérisé en ce que** la concentration de l'adjuvant chaotropique dans l'étape (b) est d'environ 8 M, la température dans l'étape (b) est d'environ 40°C, le pH dans l'étape (b) est d'environ 10,2, le pH dans l'étape (c) est d'environ 10,6, et dans l'étape (c) la quantité d'eau donne une dilution de la concentration de la cystéine ou du chlorhydrate de cystéine dans le mélange d'environ 2,5 à 3,0 mM et une concentration de l'adjuvant chaotropique de 0,5 M.

13. Procédé selon l'une ou plusieurs des revendications 1 à 12, **caractérisé en ce que** le précurseur des insulines ou des dérivés de l'insuline comporte la séquence selon la formule générale II
R²-R¹-(B2-B29)-Y-X-Gly-(A2-A20)-R³ (II) dans laquelle
R² représente
a) un atome d'hydrogène,
b) un résidu d'acide aminé du groupe lysine (Lys) ou arginine (Arg), ou
c) un peptide ayant 2 à 45 résidus d'acides aminés, contenant le résidu d'acide aminé lysine (Lys) ou arginine (Arg) à l'extrémité carboxyle du peptide,
R¹ est un résidu de phénylalanine (Phe) ou une liaison covalente,
(B2-B29) représente les résidus d'acides aminés sur les positions B2 à B29 de la chaîne B de l'insuline humaine, de l'insuline animale ou d'un dérivé de l'insuline ayant éventuellement subi des variations sur ou plusieurs de ses positions,
Y représente un résidu d'acide aminé génétiquement codable,
X représente
a) un résidu d'acide aminé du groupe histidine (His), lysine (Lys) ou arginine (Arg) ou
b) un peptide ayant 2 à 35 résidus d'acides aminés, contenant le résidu d'acide aminé lysine (Lys) ou arginine (Arg) à l'extrémité N-terminale et à l'extrémité carboxyle du peptide, ou
c) un peptide ayant 2 à 35 acides aminés génétiquement codables, contenant 1 à 5 résidus d'histidine,
(A2-A20) représente les résidus d'acides aminés sur les positions A2 à A20 de la chaîne B de l'insuline humaine, de l'insuline animale ou d'un dérivé de l'insuline ayant éventuellement subi des variations sur ou plusieurs de ses positions, et
R³ représente un résidu d'acide aminé génétiquement codable.

14. Procédé selon la revendication 13, **caractérisé en ce que**, dans la formule II
R² représente
a) un atome d'hydrogène, ou
b) un peptide ayant 2 à 25 résidus d'acides aminés, contenant le résidu d'acide aminé arginine (Arg) à l'extrémité carboxyle du peptide,
R¹ représente un résidu de phénylalanine (Phe),
(B2-B29) représente les résidus d'acides aminés sur les positions B2 à B29 de la chaîne B de l'insuline humaine,
Y représente un résidu d'acide aminé du groupe alanine (Ala), thréonine (Thr) ou sérine (Ser),
X représente le résidu d'acide aminé arginine (Arg) ou un peptide ayant la séquence d'acides aminés de la chaîne C de l'insuline humaine,
(A2-A20) représente les résidus d'acides aminés sur les positions A2 à A20 de la chaîne B de l'insuline humaine, et
R³ représente un résidu d'acide aminé du groupe asparagine (Asn), sérine (Ser) ou glycine (Gly).

15. Procédé selon la revendication 13, **caractérisé en ce que**, dans la formule II,
R² représente
a) un atome d'hydrogène ou
b) un peptide ayant 2 à 15 résidus d'acides aminés, à l'extrémité carboxyle duquel se trouve un résidu d'arginine (Arg),
R¹ est un résidu de phénylalanine (Phe),
(B2-B29) représente les résidus d'acides aminés sur les positions B2 à B29 de la chaîne B de l'insuline humaine,
Y représente un résidu de thréonine (Thr),
X le résidu d'acide aminé arginine (Arg) ou un peptide ayant 2 à 35 résidus d'acides aminés, deux résidus d'acides aminés basiques, en particulier l'arginine (Arg) et/ou la lysine (Lys) se trouvant au début et à la fin du peptide,
(A2-A20) représente les résidus d'acides aminés sur les positions A2 à A20 de la chaîne B de l'insuline humaine, et
R³ représente le résidu d'acide aminé asparagine (Asn) ou glycine (Gly).
